# EUROPEAN PATENT APPLICATION

(11) **EP 3 406 182 A2**
(43) Date of publication of application: **28.11.2018**
(21) Application number: 18174271.9
(22) Date of filing: 25.05.2018
(51) Int. Cl.: A61B 1/00, A61B 1/267

(54) **BRONCHOSCOPY SYSTEMS AND COUPLING DEVICES THEREOF**

(30) Priority: 26.05.2017 US 201715606193
(71) Applicant: Covidien LP, Mansfield, MA 02048 (US)
(72) Inventor: KRIMSKY, William S., Forest Hill, MD 21050 (US); STOPEK, Joshua B., Minneapolis, MN 55419 (US)
(74) Representative: Maschio, Antonio

(57) **Abstract**

A coupling device for use with a bronchoscope includes a first portion and a second portion extending laterally from the first portion. The first portion defines a first passageway therethrough configured for receipt of a bronchoscope. The second portion defines a second passageway therethrough configured for receipt of a surgical instrument. The first passageway defines a first axis and the second passageway defines a second axis that is parallel with the first axis.

## Description

### BACKGROUND

### Technical Field

The present disclosure relates to rigid or surgical instruments and, more specifically, to coupling devices for coupling an endoscope and an auxiliary surgical instrument.

### Description of Related Art

A common interventional procedure in the field of pulmonary medicine is bronchoscopy, in which a bronchoscope is inserted into the airways through the patient's nose or mouth. Bronchoscopes are routinely used in the diagnosis and treatment of lung conditions, such as, lung cancer, airway stenosis, emphysema, etc.

The structure of a bronchoscope generally includes a handle and a long, thin, flexible tube extending from the handle. The tube typically defines a lumen or working channel therethrough for the insertion of instruments, such as, for example, diagnostic tools (e.g., biopsy tools, etc.) or therapeutic tools (e.g., lasers, cryogenic probes, radio frequency probes, microwave tissue treatment probes, etc.). A steering mechanism of the bronchoscope may be used to effect a deflection of a distal tip of the bronchoscope tube in one or more directions such that the distal tip of the bronchoscope may be maneuvered and approximated toward target tissue.

Typically, during a procedure, a clinician holds the bronchoscope handle with one hand and the bronchoscope tube with the other hand, and manipulates the distal tip of the bronchoscope inside the lung by rotating a deflection lever of the handle and by pushing and pulling the tube of the bronchoscope. Once the distal tip is disposed adjacent target tissue, an instrument may be inserted into the working channel of the bronchoscope to perform a diagnostic or therapeutic procedure. In some situations, an extendable working channel ("EWC") is inserted into and through the working channel of the bronchoscope. The EWC has a smaller diameter than the bronchoscope tube permitting access to more remote areas of the lung (e.g., the periphery of the lung), and defines a working channel or lumen therethrough for the passage of instruments.

The EWC is limited in the number of surgical instruments it can accommodate, thus requiring the removal of one surgical instrument from the EWC prior to using another surgical instrument. Accordingly, there is a need for the ability to use additional surgical instruments with the bronchoscope when the lumen or lumens of the bronchoscope are occupied. Additionally, some telescopes (e.g., a particular type of bronchoscope) without internal lumens can be coupled to in a similar manner.

### SUMMARY

Provided in accordance with the present disclosure is a coupling device for use with a bronchoscope. The coupling device includes a first portion and a second portion extending laterally from the first portion. The first portion defines a first passageway therethrough configured for receipt of a flexible scope. The second portion defines a second passageway therethrough configured for receipt of a surgical instrument. The first passageway defines a first axis and the second passageway defines a second axis that is parallel with the first axis.

In some embodiments, the first passageway may be configured to secure a bronchoscope therein and the second passageway may be configured to permit sliding of a surgical instrument therein.

It is contemplated that the first portion may have an inner surface that defines the first passageway, and the second portion may have an inner surface that defines the second passageway. The inner surface of the first portion may be fabricated from a more pliable material than the inner surface of the second portion.

It is envisioned that the inner surface of the first portion may be fabricated from an elastomer and that the inner surface of the second portion may be fabricated from a lubricious material.

In some embodiments, the first passageway may have a first diameter and the second passageway may have a second diameter that is smaller than the first diameter.

It is contemplated that the first and second passageways may be cylindrical. In embodiments, the passageways may be collapsible.

It is envisioned that the coupling device may further include a sensor attached to the second portion.

In some embodiments, the coupling device may be dimensioned for passage through airways of a lung.

It is contemplated the first passageway may be resiliently biased toward a closed state, and be configured to expand to an opened state upon receipt of the bronchoscope.

It is envisioned that the first passageway may be transitionable between an expanded state in which the first passageway assumes a diameter configured for passage of the bronchoscope, and a collapsed state in which the first passageway is closed.

Provided in accordance with the present disclosure is a surgical system for use in a flexible or rigid endoscopy and specifically for bronchoscopy. The surgical system includes a first coupling device and a second coupling device. The first coupling device includes a first portion and a second portion extending laterally from the first portion. The first portion defines a first passageway therethrough configured for receipt of a bronchoscope. The second portion defines a second passageway therethrough configured for receipt of a surgical instrument. The first passageway defines a first axis and the second passageway defines a second axis that is parallel with the first axis. The second coupling device includes a third portion and a fourth portion extending laterally from the third portion. The third portion defines a third passageway therethrough configured for receipt of a bronchoscope. The fourth portion defines a fourth passageway therethrough configured for receipt of a surgical instrument. The third passageway defines a third axis and the fourth passageway defines a fourth axis that is parallel with the third axis.

In some embodiments, the first passageway of the first coupling device and the third passageway of the second coupling device may have the same diameter. The surgical system may further include a bronchoscope, wherein the first axis of the first coupling device and the third axis of the second coupling device are configured to be aligned such that the bronchoscope is received through each of the first and third passageways.

It is contemplated that the first passageway of the first coupling device may have a first diameter configured for receipt of a first bronchoscope, and the third passageway of the second coupling device may have a second diameter configured for receipt of a second bronchoscope having a larger outer diameter than the first bronchoscope.

It is envisioned that the surgical system may further include a bronchoscope fixedly disposed within the first passageway of the first coupling device and the third passageway of the second coupling device. The surgical system may also include a surgical instrument slidably disposed within the second passageway of the first coupling device and the fourth passageway of the second coupling device. The surgical instrument may be a cannula defining a channel longitudinally therethrough.

In some embodiments, the surgical system may further include a bronchoscope fixedly disposed within the first passageway of the first coupling device and the third passageway of the second coupling device such that the first axis of the first coupling device is aligned with the third axis of the second coupling device. The surgical system may also include a first surgical instrument slidably disposed within the second passageway of the first coupling device, and a second surgical instrument slidably disposed within the fourth passageway of the second coupling device such that the second axis of the first coupling device is offset from the fourth axis of the second coupling device.

It is contemplated that the first portion of the first coupling device may have an inner surface that defines the first passageway, and that the second portion of the first coupling device may have an inner surface that defines the second passageway. The inner surface of the first portion may be fabricated from a more pliable material than the inner surface of the second portion.

It is envisioned that each of the first passageway of the first coupling device and the third passageway of the second coupling device may have a first diameter, and each of the second passageway of the first coupling device and the fourth passageway of the second coupling device may have a second diameter that is smaller than the first diameter.

In some embodiments, the surgical system may further include a control device and a surgical instrument configured for slidable receipt within the second passageway of the first coupling device. Each of the first coupling device and the surgical instrument may include a sensor in communication with the control device such that a position of both the coupling device and the surgical instrument are displayed on the control device.

Further details and aspects of exemplary embodiments of the present disclosure are described in more detail below with reference to the appended figures.

As used herein, the terms parallel and perpendicular are understood to include relative configurations that are substantially parallel and substantially perpendicular up to about + or - 10 degrees from true parallel and true perpendicular.

### BRIEF DESCRIPTION OF THE DRAWINGS

Various aspects and features of the present disclosure are described hereinbelow with references to the drawings, wherein:
FIG. 1 is a perspective view of a bronchoscopy system in accordance with the present disclosure;
FIG. 2 is a perspective view of a coupling device for use with a bronchoscope of the bronchoscopy system of FIG. 1;
FIG. 3 is a front view of the coupling device of FIG. 2 shown coupling the bronchoscope with a surgical instrument of the bronchoscopy system of FIG. 1;
FIG. 4 is a front view of first and second coupling devices shown coupling the bronchoscope and surgical instrument of the bronchoscopy system of FIG. 1;
FIG. 5 is a top view of first and second coupling devices shown coupling the bronchoscope and surgical instrument of the bronchoscopy system of FIG. 1; and
FIG. 6 is a perspective view of another embodiment of a coupling device for use with the bronchoscope of the bronchoscopy system of FIG. 1.

### DETAILED DESCRIPTION

The present disclosure is directed to coupling devices with or without sensors for coupling a diagnostic or therapeutic tool, e.g., a telescope such as a bronchoscope, to a surgical instrument. As will be described in detail below, the coupling device may have two conduits that are oriented side by side, wherein one of the conduits fixes a distal portion of a bronchoscope therein and another of the two conduits allows for movement of a surgical instrument relative to and through the coupling device. Accordingly, distal portions of the bronchoscope and the surgical instrument are maintained in close relation to one another inside the surgical site, for example, in a lung of a patient.

Embodiments of the present disclosure will now be described in detail with reference to the drawings in which like reference numerals designate identical or corresponding elements in each of the several views. As is understood in the art, the term "clinician" refers to a doctor, a physician, a nurse, a bronchoscopist, or any other care provider or support personnel. Further, as is understood in the art the term "proximal" refers to the portion of the surgical system including the coupling devices thereof, or any component thereof, that is closest to the clinician and the term "distal" refers to the portion of the surgical system including the coupling devices thereof, or any component thereof, that is furthest from the clinician.

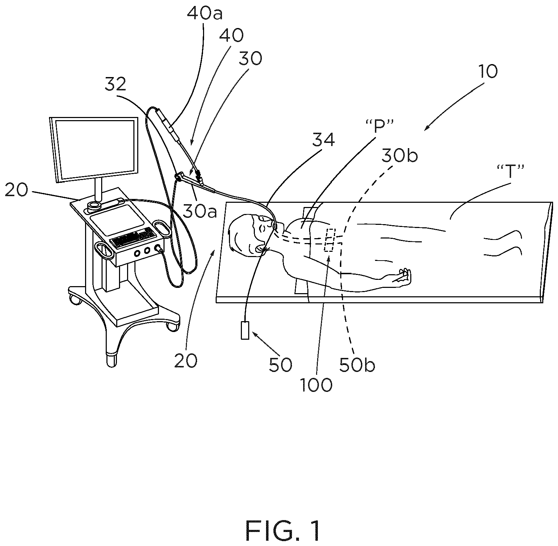

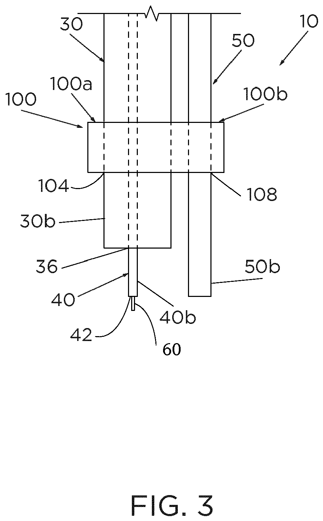

With reference to FIG. 1, a surgical system 10 for use in a bronchoscopy generally includes a control device 20, a bronchoscope 30, an extended working channel ("EWC") 40, a surgical instrument (e.g., a catheter, a stapler, an ablation instrument, an ultrasonic tool, a biopsy tool, etc.) 50, and a coupling device 100 for coupling a distal portion 30b (FIG. 3) of the bronchoscope 30 with a distal portion 50b of the surgical instrument 50. The bronchoscope 30 is electrically connected to the control device 20 (e.g., a computer), which can be used to control the operations of the bronchoscope 30. The control device 20 may receive information from the bronchoscope 30, for example, images of the surgical site, and send information to the bronchoscope 30, for example, a command to capture an image of the surgical site.

The EWC 40 is selectively insertable into and through a working channel 36 (FIG. 3) of the bronchoscope 30. A proximal portion 40a of the EWC 40 extends proximally of a proximal portion 30a of the bronchoscope 30. The surgical system 10 may further include an additional surgical instrument, such as, for example, a microwave ablation device 60 (FIG. 3) that is selectively insertable into and through a working channel 42 of the EWC 40.

In an exemplary procedure, a patient "P" is positioned on a procedure table "T" permitting a clinician to insert the distal portion 30b of the bronchoscope 30 into a natural opening (e.g., the mouth) or artificial incision in the patient. Through articulation of a control mechanism 32 of the bronchoscope 30, and pushing or pulling of a flexible insertion tube 34 of the bronchoscope 30, the distal portion 30b of the bronchoscope 30 may be directed and steered within the patient towards target tissue. Once the distal portion 30b of the bronchoscope 30 is brought into proximity to target tissue, or alternatively, once the distal portion 30b of the bronchoscope 30 is no longer capable of traversing through the airway of the patient (e.g., due to the dimensions of the bronchoscope 30 exceeding that of the airway or where a non-visual approach is preferred), the EWC 40 may be utilized to extend the reach of the bronchoscope 30 to allow access to remote areas of the lung.

In particular, the EWC 40 is inserted through the working channel 36 of the bronchoscope 30 and translated distally such that the distal portion 40b of the EWC 40 extends distally of the distal portion 30b of the bronchoscope 30. A surgical instrument, for example, the microwave ablation device 60 may be passed through the EWC 40 and into the surgical site to treat the target tissue. Additionally, the surgical instrument 50 may be inserted through the opening in the patient into the target tissue to treat the target tissue, perform a diagnostic procedure on the target tissue, or provide any other suitable function depending on the type of interventional surgical instrument being used.

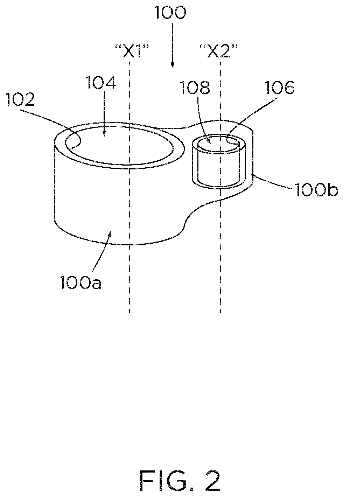

With reference to FIGS. 2 and 3, the coupling device 100 of the surgical system 10 is used with the bronchoscope 30 to couple any suitable surgical instrument 50 (e.g., a catheter, cannula, access device, biopsy tool, etc.) to the distal portion 30b of the bronchoscope 30. The coupling device 100 may be dimensioned to be passed into various airways of the lung. In some embodiments, the coupling device 100 may be dimensioned to be passed through the larger airways, such as, for example, the larynx, the trachea, and/or the bronchi. The coupling device 100 generally includes a first portion or body 100a and a second portion or body 100b extending laterally from the first portion 100a. The first and second portions 100a, 100b may be integrally connected to, or monolithically formed with, one another. In other embodiments, the first and second portions 100a, 100b of the coupling device 100 are detachably connected to one another.

The first portion 100a of the coupling device 100a has an annular inner surface 102 that defines a first passageway or channel 104. The first passageway 104 has a generally cylindrical shape configured for secure receipt of a bronchoscope, such as, for example, bronchoscope 30. In some embodiments, instead of being annular, the inner surface 102 of first portion 100a may assume any suitable shape, for example, rectangular, star-shaped, triangular, undulating, or the like, thus giving first passageway 104 a corresponding shape. The first passageway 104 may be collapsible such that until an instrument is placed therein, the first passageway 104 has a negligible to no diameter. Additionally, once an instrument is withdrawn from the first passageway 104, the diameter of the first passageway 104 automatically returns to its closed state. In some embodiments, it is contemplated that the passageway may include a valve such as a duckbill valve to facilitate this opening and closing of the first passageway 104.

The first passageway 104 defines a first axis "X1" that is coaxial with a longitudinal axis of the bronchoscope 30 when the bronchoscope 30 is disposed within the first portion 100a. The inner surface 102 of the first portion 100a is dimensioned to fit over distal portion 30b of the bronchoscope 30 while inhibiting movement of the bronchoscope 30 relative to the coupling device 100. In some embodiments, the first portion 100a may be in the form of a C-clip configured to capture an outer surface of the bronchoscope 30 such that the first portion 100a of the coupling device 100 snap-fittingly engages the bronchoscope 30.

The first passageway 104 of the first portion 100a may have a diameter of between about 0.25 mm and about 5 mm such that first passageway 104 of first portion 100a accommodates and secures a flexible bronchoscope used for viewing smaller airways (e.g., bronchioles of the lung). The first passageway 104 of the first portion 100a may have a diameter of between about 0.5 mm and about 1 mm. In some embodiments, the first passageway 104 of the first portion 100a may have a diameter of between about 5 mm and about 15 mm such that the first passageway 104 of the first portion 100a accommodates and secures a rigid bronchoscope used for viewing larger airways (e.g., the bronchi). The first passageway of the first portion 100a may have a diameter of between about 5 mm and about 6 mm. In embodiments, the first portion 100a of the coupling device 100 may include a tightening or locking mechanism (not shown), such as, for example, a lever or a screw member, that can be used to selectively adjust the diameter of the first portion 100a.

The inner surface 102 of the first portion 100a is fabricated from a pliable, high-friction material to assist in inhibiting or preventing movement of a bronchoscope within and relative to the first passageway 104. For example, the inner surface 102 of the first portion 100a may be fabricated from an elastomer. In some embodiments, the inner surface 102 of the first portion 100a may be fabricated from acetal, nylon, polyphthalamide, polyetheretherketone, or the like. First portion 100a may include a plurality of protuberances (not shown) extending from the inner surface 102 into the first passageway 104 to assist in securing a bronchoscope in the first passageway 104. It is contemplated that the inner surface 102 of the first portion 100a may be fabricated from a material capable of compressing. As such, upon the first passageway 104 receiving a bronchoscope, the diameter of the first passageway 102 expands to receive the bronchoscope, thereby enhancing the frictional engagement between the bronchoscope and the first portion 100a.

The inner surface 104 of the first portion 100a may be removable from the first passageway 104 and exchanged with a different inner surface. As such, the surgical system 10 may include a plurality of different inner surfaces in the form of tubular linings to be inserted within the first passageway 104. The linings may each have a different thickness, thus providing a clinician with the capability to adjust the diameter of the first passageway 102 so that the first portion 100a may accommodate bronchoscopes of various sizes. In other embodiments, the first portion 100a may be one unitary piece fabricated from the same material throughout, for example, a medical-grade rubber.

With continued reference to FIGS. 2 and 3, the second portion 100b of the coupling device 100 extends laterally from the first portion 100a of the coupling device 100 and is fixed to the first portion 100a. In some embodiments, the second portion 100b may be detachable from first portion 100a. The second portion 100b of the coupling device 100 has an annular inner surface 106 that defines a second passageway or channel 108 therethrough. The second passageway 108 has a generally cylindrical shape configured for receipt of a surgical instrument, such as, for example, a catheter, a forceps, a surgical stapler, a biopsy device, a cleaning device for the bronchoscope, an aspirator, an access device, an ultrasonic tool, an ablation instrument, etc. In some embodiments, instead of being annular, the inner surface 106 of the second portion 100b may assume any suitable shape, for example, rectangular, star-shaped, triangular, undulating, or the like, thus giving second passageway 108 a corresponding shape. The second passageway 108 defines a second axis "X2" that is parallel to and laterally spaced from the first axis "X1" of the first passageway 104. The inner surface 106 of the second portion 100b is dimensioned to fit over the distal portion 50b of the surgical instrument 50 while allowing for slidable or translatable movement of the surgical instrument 50 along the second axis "X2" relative to the coupling device 100.

The second passageway 108 of the second portion 100b has a diameter that is less than the diameter of the first passageway 104. As such, the second passageway 108 is dimensioned to receive surgical instruments that are typically smaller in diameter than a bronchoscope. For example, the diameter of the second passageway 108 may be between about 0.25 mm and about 1.5 mm such that the second passageway 108 of the second portion 100b slidably accommodates small surgical instruments (e.g., a needle used in fine needle aspiration) capable of gaining access to the smaller, peripheral airways of the lungs. The diameter of the second passageway 108 may between about 0.5 mm and about 1 mm. In some embodiments, the second passageway 108 of the second portion 100b may have a diameter of between about 1 mm and about 2 mm such that the second passageway 108 of the second portion 100b slidably accommodates large surgical instruments (e.g., a needle used in core needle biopsy) better suited for procedures isolated to the larynx, trachea, or bronchi. In other embodiments, the second passageway 108 of the second portion 100b may have a diameter that is more than the diameter of the first passageway 104.

The inner surface 106 of the second portion 100b is fabricated from a lower friction and more rigid material than the inner surface 102 of the first portion 100a. In particular, the inner surface 106 of the second portion 100b is fabricated from a low-friction material to facilitate slidable movement of a surgical instrument along the second axis "X2" of the second passageway 108 and relative to the coupling device 100. For example, the inner surface 106 of the second portion 100b may be fabricated from a lubricious material including, but not limited to, polytetrafluoroethylene, perfluoroalkoxy, or fluorinated ethylene propylene. The inner surface 102 of the first portion 100a may be in the form of a coating or lining.

The inner surface 102 of the second portion 100b may be removable from the second passageway 108 and exchanged with a different inner surface. As such, the surgical system 10 may include a plurality of different inner surfaces in the form of tubular linings to be inserted within the second passageway 108. The linings may each have a different coefficient of friction, allowing a clinician to affect the resistance to sliding of a surgical instrument through the second passageway 108 depending on the particular lining used. Additionally, or alternatively, the different linings for the second portion 100b may each have a different thickness, allowing a clinician to adjust the diameter of the second passageway 108 so that the second portion 100b may accommodate surgical instruments of various sizes. In other embodiments, the second portion 100b may be one unitary piece fabricated from the same material throughout, for example, polyethylene, high density polyethylene, or polyvinyl chloride.

In operation, the distal portion 30b of the bronchoscope 30 is positioned within the first passageway 104 of the first portion 100a of the coupling device 100 and forced through the first passageway 104 to overcome the static friction of the inner surface 102 of the first portion 100a. The distal portion 30b of the bronchoscope 30 may be wetted or lubricated to facilitate insertion into the first passageway 104 of the coupling device 100. In other embodiments in which the coupling device 100 includes the locking mechanism (noted above), the locking mechanism may be used to increase (or decrease) the diameter of the first passageway 104 of the coupling device prior to insertion of the distal portion 30b of the bronchoscope 30 therein. The inner surface 102 of the first portion 100a of the coupling device 100a fixes the distal portion 30b of the bronchoscope 30 thereto such that relative movement between the coupling device 100 and the bronchoscope 30 is inhibited. A distal portion of a surgical instrument, for example, the distal portion 50b of the biopsy tool 50, is positioned within the second passageway 108 of the coupling device 100, thereby coupling the bronchoscope 30 and the biopsy tool 50.

The coupling device 100, having the respective distal portions 30b, 50b of the bronchoscope 30 and the biopsy tool 50 extending therethrough, is guided into the airways of a patient. The coupling device 100 may be guided through the larynx, trachea, or bronchi of the lungs. Upon positioning the bronchoscope 30, with the coupling device 100, at the target location in the lung, the biopsy tool 50 may be manipulated by a clinician to move the biopsy tool 50 relative to the coupling device 100 and the bronchoscope 30 along the second axis "X2" of the coupling device 100 to position the working end 30b of the biopsy tool 30 at the target location.

In some embodiments, prior to positioning the biopsy tool 50 in the second passageway 108 of the coupling device 100, an extended working channel (e.g., a catheter, cannula, or access device) may be positioned in the second passageway 108. With the extended working channel disposed within the second passageway 108 of the coupling device 100 (either fixedly or slidably disposed therein), another surgical instrument, such as, for example, the biopsy tool 50, or fluid such as a lavage fluid, may be passed through the extended working channel into the surgical site.

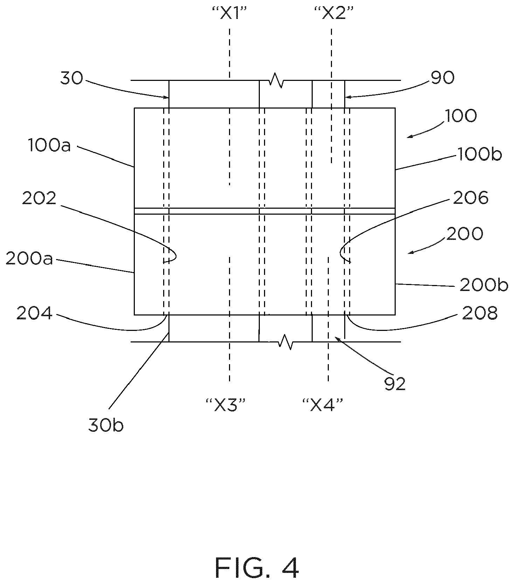

With reference to FIG. 4, the surgical system 10 may include an additional coupling device 200, similar to the coupling device 100 described above with reference to FIGS. 2 and 3. It is contemplated that the surgical system 10 may include more than two coupling devices. The second coupling device 200 assists in maintaining a surgical instrument, such as, for example, a catheter 90 or tool 50 and the bronchoscope 30 parallel with and adjacent to one another during a surgical procedure. In some embodiments, a plurality of coupling devices may be positioned along a portion or an entirety of the length of the surgical instrument 90 and the bronchoscope 30. The catheter 90 defines a channel 92 longitudinally therethrough configured for receipt of a surgical instrument, such as, for example, the biopsy tool 50 (FIG. 3).

The second coupling device 200 includes a third portion 200a and a fourth portion 200b coupled to the third portion 200a. The third portion 200a includes an annular inner surface 202 that defines a third passageway 204 therethrough. The third passageway 304 of the second coupling device 300, similar to the first passageway 104 of the first coupling device 100, is dimensioned for secure receipt of a bronchoscope, for example, the bronchoscope 30. The fourth portion 200b of the second coupling device 200 includes an annular inner surface 206 that defines a fourth passageway 208 therethrough. The fourth passageway 208 of the second coupling device 20, similar to the second passageway 108 of the first coupling device 100, is dimensioned for slidable receipt of a surgical instrument, such as, for example, the catheter 90.

The third passageway 204 defines a third axis "X3" and the fourth passageway 208 defines a fourth axis "X4" that is parallel with the third axis "X3" of the third passageway 204. In some embodiments, the third passageway 204 of the second coupling device 200 may have a larger diameter than the first passageway 104 of the first coupling device 100 so as to be able to accommodate a larger bronchoscope than the first passageway 104 of the first coupling device 100.

In operation, the first and second coupling devices 100, 200 are positioned relative to one another to align the first and second passageways 104, 108 of the first coupling device 100 with the third and fourth passageways 204, 208 of the second coupling device 200, respectively. As such, the first axis "X1" of the first passageway 104 of the first coupling device 100 is coaxial with the third axis "X3" of the third passageway 204 of the second coupling device 200, and the second axis "X2" of the second passageway 108 of the first coupling device 100 is coaxial with the fourth axis "X4" of the fourth passageway 208 of the second coupling device 200. In this way, the bronchoscope 30 may be received through each of the first and third passageways 104, 204 of respective first and second coupling devices 100, 200, and the surgical instrument 30 may be received through each of the second and fourth passageways 108, 208 of respective first and second coupling devices 100, 200. The first and second coupling devices 100, 200 may be longitudinally spaced from one another to maintain different portions of the bronchoscope 30 and the catheter 90 in close proximity to one another. For example, the first and second coupling devices 100, 200 may be longitudinally spaced from one another between about 1 mm to about 50 mm, and in some embodiments, the first and second coupling devices 100, 200 may be in abutting engagement with one another.

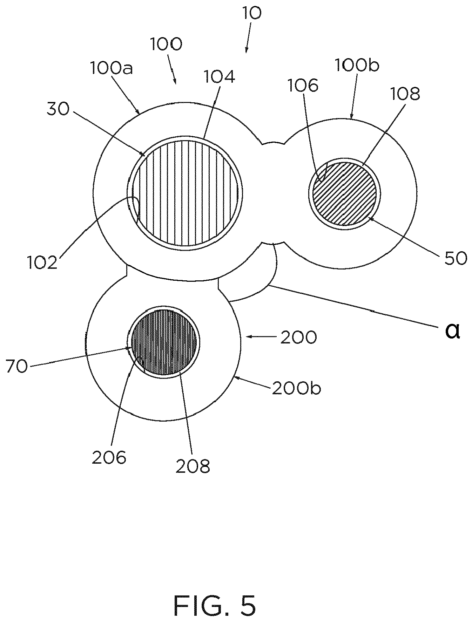

With reference to FIG. 5, instead of the first and second coupling devices 100, 200 being arranged relative to one another in the manner illustrated in FIG. 4, the first and second coupling device 100, 200 may be arranged so that only the first portion 100a of the first coupling device 100 and the third portion 200a of the second coupling device 200 overlap with one another. In particular, the first and second coupling devices 100, 200 are positioned relative to one another to align the first passageway 104 of the first coupling device 100 with the third passageway 204 of the second coupling device 200 while offsetting the second passageway 108 of the first coupling device 100 from the fourth passageway 208 of the second coupling device 200. In this orientation, the first axis "X1" of the first passageway 104 of the first coupling device 100 is coaxial with the third axis "X3" of the third passageway 204 of the second coupling device 200, and the second axis "X2" of the second passageway 108 of the first coupling device 100 is offset from the fourth axis "X4" of the fourth passageway 208 of the second coupling device 200 by an angle α (e.g., 90 degrees). In this way, the surgical system 10 can include two surgical instruments 50 and 70 for use with the coupling devices 100, 200.

The first surgical instrument 50 is received through the second passageway 108 of the first coupling device 100 and the second surgical instrument 70 is received through the fourth passageway 208 of the second coupling device 200. The bronchoscope 30 is received through each of the first and third passageways 104, 204 of respective first and second coupling devices 100, 200, similar to that shown in FIG. 4. It is contemplated that the surgical system 10 may include any number of coupling devices in an offset arrangement about the bronchoscope 30 so that any number of surgical instruments (e.g., two or more) may be coupled to the bronchoscope 30 and disposed circumferentially about the bronchoscope 30

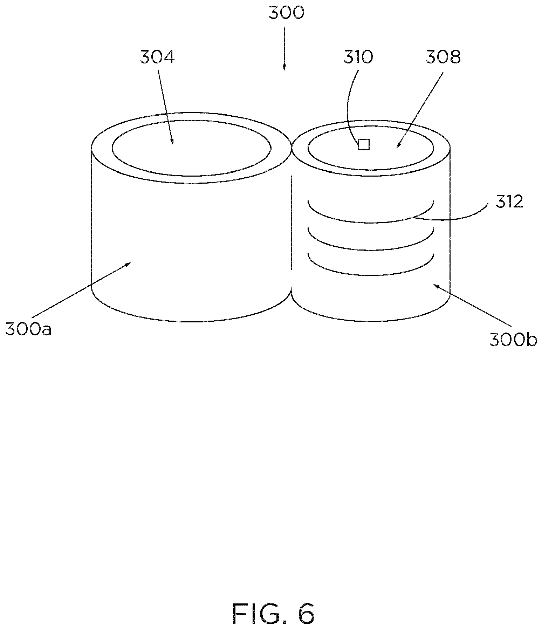

With reference to FIG. 6, another embodiment of a coupling device 300 for use with a bronchoscope is provided. The coupling device 300 is similar to the coupling devices 100, 200 described above with reference to FIGS. 1-5, and will therefore only be described in the detail necessary to elucidate any differences. The coupling device 300 has a first portion 300a and a second portion 300b extending laterally from the first portion 300a. The first portion 300a defines a first passageway 304 therethrough configured for secure receipt of a bronchoscope, such as, for example, a bronchoscope fitted with an ultrasound processor (not shown). The second portion 300b defines a second passageway 308 therethrough configured for slidable receipt of a surgical instrument, such as, for example, a fine-gauge aspiration needle (not shown).

The second portion 300b may include a sensor 310, such as, for example, electromagnetic sensors, to enhance the navigability of the coupling device 300 and/or surgical tool through airways. The sensor 310 may be in communication with the control device (FIG. 1) which provides a real-time image of the position of the sensor 310 of the coupling device 300 within the airways. The sensor 310 is incorporated into the second portion 300b of the coupling device 300. In some embodiments, the sensor 310 may be positioned on an inner or outer surface of the second portion 300b of the coupling device 300 or on or in any portion of coupling device 300. In addition to the coupling device 300 having the sensor 310, or in the alternative, the second portion 300b of the coupling device 300 may have radiopaque material 312 attached thereto. It is contemplated that the sensor 310 can be advanced relative to the scope being used and a sensor may be placed on the surgical instrument being passed through the coupling device 300 or the scope. The sensor 310 of the coupling device 300 and the sensor on the surgical instrument may be in communication with one another and with the control device (FIG. 1) such that the position of both the coupling device 300 and the surgical instrument can be projected within the system and displayed for the clinician to see.

In embodiments, the couple devices of the present disclosure may provide a clinician with the capability to cannulate different segments within a lobe or different lobes of the lung simultaneously. For example, a particular segment of a lobe may be isolated and treated, and without removing any instruments from the surgical site, an adjacent segment of the lung may also be cannulated to provide protection for the remaining area of the lung.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope and spirit of the claims appended hereto.
The invention maybe described by reference to the following numbered paragraphs:-
1. A coupling device for use with a bronchoscope, comprising:
   a first portion defining a first passageway therethrough configured for receipt of a bronchoscope, the first passageway defining a first axis; and
   a second portion extending laterally from the first portion and defining a second passageway therethrough configured for receipt of a surgical instrument, the second passageway defining a second axis that is parallel with the first axis.
2. The coupling device according to paragraph 1, wherein the first passageway is configured to secure a bronchoscope therein, and the second passageway is configured to permit sliding of a surgical instrument therein.
3. The coupling device according to paragraph 1, wherein the first portion has an inner surface that defines the first passageway, and the second portion has an inner surface that defines the second passageway, at least the inner surface of the first portion being fabricated from a more pliable material than the inner surface of the second portion.
4. The coupling device according to paragraph 3, wherein the inner surface of the first portion is fabricated from an elastomer.
5. The coupling device according to paragraph 3, wherein the inner surface of the second portion is fabricated from a lubricious material.
6. The coupling device according to paragraph 1, wherein the first passageway has a first diameter and the second passageway has a second diameter that is smaller than the first diameter.
7. The coupling device according to paragraph 1, wherein the first and second passageways are cylindrical.
8. The coupling device according to paragraph 1, further comprising a sensor attached to the second portion.
9. The coupling device according to paragraph 1, wherein the coupling device is dimensioned for passage through airways of a lung.
10. The coupling device according to paragraph 1, wherein the first passageway is resiliently biased toward a closed state, and is configured to expand to an opened state upon receipt of the bronchoscope.
11. The coupling device according to paragraph 1, wherein the first passageway is transitionable between an expanded state in which the first passageway assumes a diameter configured for passage of the bronchoscope, and a collapsed state in which the first passageway is closed.

## Claims

1. A coupling device for use with a bronchoscope, comprising:
a first portion defining a first passageway therethrough configured for receipt of a bronchoscope, the first passageway defining a first axis; and
a second portion extending laterally from the first portion and defining a second passageway therethrough configured for receipt of a surgical instrument, the second passageway defining a second axis that is parallel with the first axis.

2. The coupling device according to claim 1, wherein the first passageway is configured to secure a bronchoscope therein, and the second passageway is configured to permit sliding of a surgical instrument therein.

3. The coupling device according to claim 1 or claim 2, wherein the first portion has an inner surface that defines the first passageway, and the second portion has an inner surface that defines the second passageway, at least the inner surface of the first portion being fabricated from a more pliable material than the inner surface of the second portion.

4. The coupling device according to claim 3, wherein the inner surface of the first portion is fabricated from an elastomer.

5. The coupling device according to claim 3 or claim 4, wherein the inner surface of the second portion is fabricated from a lubricious material.

6. The coupling device according to any preceding claim, wherein the first passageway has a first diameter and the second passageway has a second diameter that is smaller than the first diameter.

7. The coupling device according to any preceding claim, wherein the first and second passageways are cylindrical.

8. The coupling device according to any preceding claim, further comprising a sensor attached to the second portion.

9. The coupling device according to any preceding claim, wherein the coupling device is dimensioned for passage through airways of a lung.

10. The coupling device according to any preceding claim, wherein the first passageway is resiliently biased toward a closed state, and is configured to expand to an opened state upon receipt of the bronchoscope.

11. The coupling device according to any preceding claim, wherein the first passageway is transitionable between an expanded state in which the first passageway assumes a diameter configured for passage of the bronchoscope, and a collapsed state in which the first passageway is closed.
